# EUROPEAN PATENT SPECIFICATION

(11) **EP 3 695 832 B1**
(45) Date of publication and mention of the grant of the patent: **18.06.2025**
(21) Application number: 20157498.5
(22) Date of filing: 14.02.2020
(51) Int. Cl.: A61K 31/095, A61K 31/353, A61K 31/506, A61K 38/39, A61P 17/14

(54) **COMPOSITION FOR TREATING HAIR LOSS**
ZUSAMMENSETZUNG ZUR VERMINDERUNG VON HAARAUSFALL
COMPOSITION POUR TRAITER LA CHUTE DE CHEVEUX

(30) Priority: 14.02.2019 GB 201902054
(43) Date of publication of application: 19.08.2020
(73) Proprietor: DUI Clinique AS, 3111 Tønsberg (NO)
(72) Inventor: MORTENSEN, Nils K., Tønsberg 3111 (NO); MORTENSEN, Audrey Wong, Tønsberg 3111 (NO)
(74) Representative: Dehns

(56) References cited:
- EP-A1- 2 280 684
- CN-A- 102 772 324
- CN-A- 107 812 171
- JP-A- H03 287 516
- US-B2- 9 445 979
- H. GALBRAITH: "In vitro methodology, hormonal and nutritional effects and fibre production in isolated ovine and caprine anagen hair follicles", ANIMAL, vol. 4, no. 09, 7 January 2010 (2010-01-07), GB, pages 1482 - 1489, XP055258073, ISSN: 1751-7311, DOI: 10.1017/S1751731109991595
- HENGL THOMAS ET AL: "Cystine-thiamin-containing hair-growth formulation modulates the response to UV radiation in anin vitromodel for growth-limiting conditions of human keratinocytes", JOURNAL OF PHOTOCHEMISTRY AND PHOTOBIOLOGY B: BIOLOGY, ELSEVIER SCIENCE S.A., BASEL, CH, vol. 189, 17 September 2018 (2018-09-17), pages 318 - 325, XP085552954, ISSN: 1011-1344, DOI: 10.1016/J.JPHOTOBIOL.2018.09.005
- CETINKUNAR D ET AL: "Evaluation of the effects of "millet extract, wheat germ oil, calcium pantothenate and L-cystine combination" therapy in women with diffuse alopecia by using "digital phototrichogram"", DERI HASTALIKLARI VE FRENGI ARSIVI; ; ARCHIVES OF THE TURKISH DERMATOLOGY AND VENEREOLOGY, GALENOS YAYINEVI, TR, vol. 46, no. 2, 1 January 2012 (2012-01-01), pages 78 - 83, XP009521301, ISSN: 1019-214X, DOI: 10.4274/TURKDERM.48295
- GOMEZ GRAU E ET AL: "Effectiveness of a dietary supplement with serenoa serrulata and tocotrienol-tocopherol against female androgenetic alopecia and telogen effluvium. Report of a pilot study", vol. 96, no. 1, 1 January 2015 (2015-01-01), XP009521297, ISSN: 0325-2787, Retrieved from the Internet <URL:https://www.researchgate.net/publication/282176915_Effectiveness_of_a_dietary_supplement_with_serenoa_serrulata_and_tocotrienol-tocopherol_against_female_androgenetic_alopecia_and_telogen_effluvium_Report_of_a_pilot_study>
- SANT'ANNA ADDOR F A DONATO L C ET AL: "Comparative evaluation between two nutritional supplements in the improvement of telogen effluvium", vol. 11, 1 January 2018 (2018-01-01), pages 431 - 436, XP009521293, ISSN: 1178-7015, Retrieved from the Internet <URL:https://www.ncbi.nlm.nih.gov/pmc/articles/doi/10.2147/CCID.S173082> DOI: 10.2147/CCID.S173082
- DATABASE MEDLINE [online] US NATIONAL LIBRARY OF MEDICINE (NLM), BETHESDA, MD, US; August 2017 (2017-08-01), PATEL DEEPA P ET AL: "A Review of the Use of Biotin for Hair Loss.", Database accession no. NLM28879195
- PATEL DEEPA P ET AL: "A Review of the Use of Biotin for Hair Loss.", SKIN APPENDAGE DISORDERS AUG 2017, vol. 3, no. 3, August 2017 (2017-08-01), pages 166 - 169, ISSN: 2296-9195
- ANONYMOUS: "Grape seed procyanidin B2 protects podocytes from high glucose-induced mitochondrial dysfunction and apoptosis via the AMPK-SIRT1-PGC-1[alpha] axis in vitro - PubMed", 1 January 2021 (2021-01-01), pages 1 - 1, XP093118275, Retrieved from the Internet <URL:https://pubmed.ncbi.nlm.nih.gov/26650960/> [retrieved on 20240111]

## Description

### Field of the invention

The invention relates to a composition for retarding hair loss or facilitating hair growth or regrowth. The invention also relates to the non-therapeutic use of the composition as a hair growth or regrowth product, and to the composition for use in the treatment or prevention of hair loss.

### Background to the invention

Hair loss can be caused by a number of factors. Hair loss can be genetic (e.g. male pattern baldness), or can be caused by various other factors such as an illness, stress, cancer treatment, weight loss or iron deficiency. There are various types of hair loss such as male-pattern hair loss, female-pattern hair loss, alopecia areata, or telogen effluvium.

The two main treatments for male pattern baldness are minoxidil and finasteride. Minoxidil can also be used to treat female pattern baldness. These treatments, however, do not work for everyone and can be expensive. Furthermore, common side effects for minoxidil include burning or irritation of the eye, itching, redness or irritation at the treated area, and unwanted hair growth elsewhere on the body. The use of finasteride has been associated with sexual function side effects.

Other hair loss treatments include steroid injections, steroid creams, immunotherapy, light treatment, tattooing, hair transplants, scalp reduction surgery, artificial hair transplants. Many of those treatments have disadvantages in terms of cost, effectiveness, side-effects and/or invasiveness. Document: HENGL THOMAS ET AL: "Cystine-thiamin-containing hair-growth formulation modulates the response to UV radiation in an in vitro model for growth-limiting conditions of human keratinocytes",JOURNAL OF PHOTOCHEMISTRY AND PHOTOBIOLOGY B: BIOLOGY, ELSEVIER SCIENCE S.A., BASEL, CH, vol. 189, 17 September 2018 (2018-09-17), pages 318-325,
discloses that an oral formulation containing I -cystine, thiamin, calcium d -pantothenate, medicinal yeast, keratin and p-aminobenzoic acid (Panto[vi]gar^{®}) has demonstrated clinical efficacy for the treatment of diffuse telogen effluvium; In clinical practice, a combination of I -cystine, thiamin, calcium d -pantothenate, yeast, p-aminobenzoic acid (PABA) and keratin has been successfully used to treat diffuse hair loss.

It would clearly be desirable to provide a composition for retarding hair loss or facilitating hair growth/regrowth, in particular on the human scalp, by using a non-invasive, cost-efficient treatment with few or no side effects.

The present invention addresses at least some of the above issues. The particular combination of ingredients in the composition is beneficial in that hair (re-)growth is enhanced, yet the ingredients are unharmful, cheap, and the composition can simply be ingested daily, without the need for any invasive procedures.

### Summary of the invention

In a first aspect, the present invention provides a composition comprising:
i) Riboflavin (vitamin B2) and Biotin (vitamin B7);
ii) cysteine;
iii) lysine;
iv) procyanidin B-2;
v) mangosteen extract;
vi) methionine; and
vii) collagen;
viii) ascorbic acid;
ix) alpha-tocopheryl acetate;
x) zinc gluconate;
xi) ferrous fumarate;
xii) copper gluconate;
xiii) beta-carotene;
xiv) thiamine hydrochloride;
xv) nicotinamide;
xvi) D-calcium pantothenate;
xvii) pyridoxine hydrochloride;
xviii) cyanocobalamin;
xix) folic acid;
xx) L-selenmethionine; and
xxi) magnesium hydroxide carbonate;
or any pharmaceutically acceptable salts or esters thereof;
wherein the lysine is present in an amount of 5-12 wt% based on the total weight of the composition;
wherein the procyanidin B-2 is present in an amount of 0.5-15 wt% based on the total weight of the composition;
wherein the methionine is present in an amount of 0.2-3.0 wt% based on the total weight of the composition.

In a further aspect, the invention provides the non-therapeutic use of the composition as herein defined as a hair growth or regrowth product.

In a further aspect, the invention provides the composition as defined herein for use in the treatment or prevention of hair loss.

The features of the aspects and/or embodiments indicated herein are usable individually and in combination in all aspects and embodiments of the invention where technically viable, unless otherwise indicated.

### Detailed description of the invention

The present invention provides a composition for the treatment or prevention of hair loss. The compositions thus act to promote hair growth/regrowth and/or reduce the rate of hair loss.

The composition of the invention comprises
i) Riboflavin (vitamin B2) and Biotin (vitamin B7);
ii) cysteine;
iii) lysine;
iv) procyanidin B-2;
v) mangosteen extract;
vi) methionine; and
vii) collagen;
viii) ascorbic acid;
ix) alpha-tocopheryl acetate;
x) zinc gluconate;
xi) ferrous fumarate;
xii) copper gluconate;
xiii) beta-carotene;
xiv) thiamine hydrochloride;
xv) nicotinamide;
xvi) D-calcium pantothenate;
xvii) pyridoxine hydrochloride;
xviii) cyanocobalamin;
xix) folic acid;
xx) L-selenmethionine; and
xxi) magnesium hydroxide carbonate;
or any pharmaceutically acceptable salts or esters thereof;
wherein the lysine is present in an amount of 5-12 wt% based on the total weight of the composition;
wherein the procyanidin B-2 is present in an amount of 0.5-15 wt% based on the total weight of the composition;
wherein the methionine is present in an amount of 0.2-3.0 wt% based on the total weight of the composition.

As a general point, when particular vitamins are referred to herein, the term is intended to cover conventional derivatives of such vitamins (e.g. acetates), salts, solvates or hydrates thereof, where technically appropriate. For example, the term vitamin B1 would cover thiamine hydrochloride, the term vitamin B5 covers D-calcium pantothenate, the term vitamin B6 covers pyridoxine hydrochloride, and the term vitamin E covers alpha tocopheryl acetate.

Where wt% amounts are given for vitamins or aminoacids, the wt% relates to the amount of actual vitamin or actual amino acid required in the composition rather than the weight of the compound used. For example, where the composition comprises thiamine HCl, D-calcium pantothenate, pyridoxine hydrochloride, L-cysteine HCl monohydrate, or L-lysine monohydrochloride, the indicated wt% values relate to the amount of thiamine, pantothenate, pyridoxine, L-cysteine and L-lysine respectively. We do not count the weight of the inactive counter ion. The wt% values indicate the desired range of the active agent therefore.

### Vitamins

The vitamin B compounds of the invention are selected from vitamin B1, B2, B3, B5, B6, B7, B9 and B12. All of vitamins B1, B2, B3, B5, B6, B7, B9 and B12 are present in the composition.

Vitamin B1 covers thiamine or a derivative thereof such as a salt thereof, such as thiamine hydrochloride. Vitamin B2 covers riboflavin or a derivative thereof such as a salt thereof.

Vitamin B3 covers nicotinamide or a derivative thereof.

Vitamin B5 covers pantothenic acid or a derivative thereof such as a salt thereof, such as calcium pantothenate (e.g. D-calcium pantothenate), as well as pantothenol.

Vitamin B6 covers pyridoxine or a derivative thereof such as a salt thereof, such as pyridoxine hydrochloride.

Vitamin B7 covers biotin or a derivative thereof, e.g. D-biotin.

Vitamin B9 covers folic acid or a derivative thereof such as a salt thereof.

Vitamin B12 covers cyanocobalamin or a derivative thereof.

The composition comprises Riboflavin or a derivative thereof (vitamin B2) and D-Biotin or a derivative thereof (vitamin B7). Typically, the amount of riboflavin in the composition is 0.001-0.2 wt%, e.g. 0.005-0.2 wt%, 0.01-0.1 wt%, or 0.01-0.05 wt%, based on the total weight of the composition.

Typically, the amount of D-Biotin in the composition is 0.0001-0.01 wt%, e.g. 0.001-0.005 wt% based on the total weight of the composition.

Typically, component i) of the composition is present in an amount of 0.05-5.0 wt%, based on the total weight of the composition, such as 0.1-2.0 wt%, e.g. 0.2-1.0 wt%, based on the total weight of the composition.

The composition further comprises ascorbic acid (vitamin C). Preferably, the ascorbic acid is present in an amount of 0.1-5.0 wt%, e.g. 0.5-3.0 wt% or 1.0-2.0 wt%, based on the total weight of the composition.

The composition further comprises alpha-tocopheryl acetate as a vitamin E or derivative thereof. Preferably the at least one vitamin E is present in an amount of 0.05-5.0 wt%, e.g. 0.1-1.0 wt%, based on the total weight of the composition.

The composition comprises a plurality of vitamin B compounds, vitamin C, and vitamin E.

### Metal Salt(s)

The composition comprises at least one organic metal salt, wherein the metal is selected from Zn, Fe, Cu, and Mg.

The composition of the invention comprises ferrous fumarate, preferably in an amount of 0.05-5.0 wt% of Fe, e.g. 0.1-1.0 wt% of Fe, based on the total weight of the composition. The addition of iron can be beneficial as iron deficiency has been identified as a cause of hair loss.

The invention comprises zinc gluconate, ferrous fumarate, and copper gluconate.

As a source of magnesium, the composition contains magnesium hydroxide carbonate.

Preferably, the composition comprises metal salts of Zn, Fe, Cu and/or Mg, in a total amount of 0.5-10 wt%, e.g. 1-5 wt% based on the weight of metal ion(s) with respect to the total weight of the composition.

The composition comprises a Se compound (i.e. a source of selenium). Preferably, the composition comprises 0.0001-0.01 wt% Se based on the total weight of the composition. The Se compound is L-Selenomethionine.

### Amino acids and other compounds

The composition of the invention comprises cysteine or a derivative thereof. This is preferably L-cysteine or an L-cysteine derivative such as L-cysteine HCl monohydrate. The cysteine is preferably present in an amount of 0.1-10 wt%, e.g. 0.2-5.0 wt% or 0.5-3.0 wt%, based on the total weight of the composition.

The composition of the invention comprises lysine or a derivative thereof. This is preferably L-Lysine or an L-lysine salt such as L-lysine monohydrochloride. The lysine is present in an amount of 5-12 wt%, based on the total weight of the composition.

The composition of the invention comprises procyanidin B-2, such as procyanidin B-2 extracted from apples. The procyanidin is present in an amount of 0.5-15 wt%, e.g. 1-10 wt% or 2-8 wt%, based on the total weight of the composition.

The composition of the invention comprises mangostin (i.e. mangosteen extract). The mangosteen extract is preferably present in an amount of 0.1-10 wt%, e.g. 0.2-5.0 wt% or 0.5-3.0 wt%, based on the total weight of the composition.

The composition of the invention comprises methionine or a derivative thereof. This is preferably L-methionine. The methionine is present in an amount of 0.2-3.0 wt%, e.g. 0.5-2.0 wt%, based on the total weight of the composition.

### Collagen

The composition of the invention requires collagen, typically hydrolysed collagen. Preferably, the collagen is the most abundant component of the composition. Typically, the collagen is present in at least 20 wt%, e.g. at least 30 wt%, 40 wt% or 50 wt%, based on the total weight of the composition. Preferably, the collagen is present in an amount of 30-90 wt%, e.g. 40-80 wt%, 50-70 wt%, or 55-65 wt%, based on the total weight of the composition. The reference herein to 'collagen' also encompasses a mixture of two or more collagens. Thus, for example, if two different collagens are present, the amounts described above are for the mixture of collagen.

In a particular embodiment, the collagen is from fish. In a further embodiment, the collagen is Derapro-100 collagen.

### Further Additives

The composition of the invention typically comprises further additives selected from chlorophyll (e.g. E144ii), silicon dioxide (e.g. E551), flavourings (e.g. natural extract flavourings), citric acid anhydrous, sucralose, or inulin. These further additives are preferably present in a total amount of 1.0-15 wt%, based on the total weight of the composition, e.g. 5.0-10 wt% based on the total weight of the composition.

### Composition

Typically, in the composition of the invention, the sum of components i)-vi) makes up 5-50 wt% of the composition, such as 10-30 wt% of the composition.

In a further embodiment, the sum of components i)-vii) makes up 60-90 wt% of the composition, such as 65-85 wt%, e.g. 70-80 wt%.

In a further embodiment, the invention further comprises chlorophyll, silicon dioxide, natural flavourings, citric acid anhydrous, sucralose and/or inulin.

### Uses

The composition of the present invention is for use in the prevention or treatment of hair loss. The hair loss can be caused by alopecia areata, cancer treatment (e.g. chemotherapy), iron deficiency, a medication side-effect, or weight loss. The compounds of the invention can be used for clinical causes of hair loss as described above, or non-clinical causes, e.g. male-pattern hair loss (MPHL) and female-pattern hair loss (FPHL).

Essentially the composition of the invention is for retarding hair loss or facilitating hair growth/regrowth, in particular on the human scalp, by using a non-invasive, cost-efficient treatment with few or no side effects.

The composition is typically ingested daily. It may therefore be suitable for oral administration, e.g. tablet, capsule and so on. In a particular embodiment, the composition is in the form of a powder. Such a powder can be mixed with water for drinking. In a particular embodiment, the composition is to be taken at a dose of 2-10 g/day, e.g. 4-8 g/day.

### Example 1

The following composition was prepared:

| | Input (of raw ingredient) / mg | wt% | Active part / mg | |
|---|---|---|---|---|
| | | | | |
| Beta carotene, 20% | 27 | 0.45 | Beta carotene | 4.5 |
| Ascorbic acid powder | 95.61 | 1.58 | Vit C | 75 |
| DL-alpha tocopheryl acetate 50% | 46.95 | 0.78 | Vit E | 15 |
| Thiamine hydrochloride | 1.794 | 0.03 | Vit B1 | 1.2 |
| Riboflavin | 1.572 | 0.03 | Vit B2 | 1.4 |
| Nicotinamide | 17.69 | 0.30 | Vit B3 | 16 |
| D-calcium pantothenate | 9.054 | 0.15 | Vit B5 | 7.5 |
| Pyridoxine hydrochloride | 2.856 | 0.05 | Vit B6 | 2.1 |
| Cyanocobalamin (vit B12, 1.0%) | 0.63 | 0.01 | Vit B12 | 0.0045 |
| D-Biotin | 0.144 | >0.01 | Biotin | 0.11 |
| Folic acid | 0.138 | >0.01 | Vit B9 | 0.1 |
| Zinc gluconate | 103 | 1.72 | Zn | 12 |
| Ferrous fumarate | 83.08 | 1.3 | Fe | 27 |
| Copper gluconate | 8.31 | 0.13 | Cu | 1 |
| L-selenmethionine | 10.3 | 0.05 | Se | 0.05 |
| Magnesium hydroxide DC E.P. | 397.73 | 6.63 | Mg | 150 |
| L-cysteine HCL monohydrate | 144.93 | 2.4 | L-cysteine | 100 |
| L-lysine monohydrochloride | 625 | 10.41 | L-lysine | 500 |
| Procyanidin B-2 fraction extracted from apples | 300 | 5.0 | | 300 |
| Mangosteen extract | 100 | 1.67 | | 100 |
| L-methionine | 50.77 | 0.85 | L-methionine | 50 |
| Chlorphyll E144ii | 4.212 | 0.07 | Chlorophyll | 4 |
| silicon dioxide E551 | 51.55 | 0.85 | Silics | 50 |
| Derapro 100 collagen | 3500 | 58 | Collagen | 3500 |
| Natural strawberry flavour | 120 | 2.0 | | |
| Natural vanilla extract flavour | 60 | 1.0 | | |
| Citric acid anhydrous | 6 | 0.1 | | |
| sucralose | 6 | 0.1 | | |
| inulin | 225.68 | 3.75 | | |
| | | | | |
| **TOTAL** | **6000 mg** | 100 | | |

### Example 2

A 15 yr old patient suffering from alopecia areata was treated with the composition of example 1. Her hair growth returned to normal.

## Claims

1. A composition comprising
i) Riboflavin (vitamin B2) and Biotin (vitamin B7);
ii) cysteine;
iii) lysine;
iv) procyanidin B-2;
v) mangosteen extract;
vi) methionine; and
vii) collagen;
viii) ascorbic acid;
ix) alpha-tocopheryl acetate;
x) zinc gluconate;
xi) ferrous fumarate;
xii) copper gluconate;
xiii) beta-carotene;
xiv) thiamine hydrochloride;
xv) nicotinamide;
xvi) D-calcium pantothenate;
xvii) pyridoxine hydrochloride;
xviii) cyanocobalamin;
xix) folic acid;
xx) L-selenmethionine; and
xxi) magnesium hydroxide carbonate;
or any pharmaceutically acceptable salts or esters thereof;
wherein the lysine is present in an amount of 5-12 wt% based on the total weight of the composition;
wherein the procyanidin B-2 is present in an amount of 0.5-15 wt% based on the total weight of the composition;
wherein the methionine is present in an amount of 0.2-3.0 wt% based on the total weight of the composition.

2. A composition as claimed in claim 1 wherein one or more of the following requirements applies:
ii) is L-cysteine, iii) is L-lysine, and/or vi) is L-methionine, or any pharmaceutically acceptable salts or esters thereof, and in any combination thereof.

3. A composition as claimed in any preceding claim, wherein
i) comprises at least riboflavin and D-biotin, preferably i) comprises thiamine hydrochloride, riboflavin, nicotinamide, D-calcium pantothenate, pyridoxine hydrochloride, cyanocobalamin, D-Biotin and folic acid;
ii) is L-cysteine HCl monohydrate;
iii) is L-lysine monohydrochloride;
iv) is Procyanidin B-2 fraction extracted from apple;
v) is mangostin;
vi) is L-methionine; and/or
vii) is hydrolysed collagen from fish,
or any combinations thereof.

4. A composition as claimed in any preceding claim, wherein collagen component vii) is present in an amount of 40-80 wt%, preferably 50-70 wt%, based on the total weight of the composition.

5. A composition as claimed in any preceding claim, wherein the sum of components i)-vi) makes up 5-50 wt% of the composition, such as 10-30 wt% of the composition.

6. A composition as claimed in any preceding claim wherein the sum of components i)-vii) makes up 60-90 wt% of the composition, such as 65-85 wt%, e.g. 70-80 wt%.

7. A composition as claimed in any preceding claim, wherein said composition is in powder form.

8. The non-therapeutic use of a composition as claimed in claims 1-7 as a hair regrowth product.

9. A composition as claimed in claims 1-7 for use in the treatment or prevention of hair loss.

10. A composition for use as claimed in claim 9 wherein the hair loss is caused by alopecia areata, cancer treatment (such as chemotherapy), iron deficiency, a medication side-effect, or weight loss.

11. A composition for use as claimed in claim 9, comprising administering the composition to a human at a dosage of 2-10 g/day, such as 4-8 g/day.

## Patentansprüche

1. Zusammensetzung, umfassend
i) Riboflavin (Vitamin B2) und Biotin (Vitamin B7);
ii) Cystein;
iii) Lysin;
iv) Procyanidin B-2;
v) Mangostan-Extrakt;
vi) Methionin; und
vii) Kollagen;
viii) Ascorbinsäure;
ix) Alpha-Tocopherylacetat;
x) Zinkgluconat;
xi) Eisenfumarat;
xii) Kupfergluconat;
xiii) Beta-Carotin;
xiv) Thiaminhydrochlorid;
xv) Nicotinamid;
xvi) D-Calciumpantothenat;
xvii) Pyridoxinhydrochlorid;
xviii) Cyanocobalamin;
xix) Folsäure;
xx) L-Selenmethionin; und
xxi) Magnesiumhydroxidcarbonat;
oder pharmazeutisch verträgliche Salze oder Ester davon;
wobei das Lysin in einer Menge von 5-12 Gew.-% basierend auf dem Gesamtgewicht der Zusammensetzung vorliegt;
wobei das Procyanidin B-2 in einer Menge von 0,5-15 Gew.-% basierend auf dem Gesamtgewicht der Zusammensetzung vorliegt;
wobei das Methionin in einer Menge von 0,2-3,0 Gew.-% basierend auf dem Gesamtgewicht der Zusammensetzung vorliegt.

2. Zusammensetzung nach Anspruch 1, wobei eine oder mehrere der folgenden Anforderungen gelten:
ii) ist L-Cystein, iii) ist L-Lysin und/oder vi) ist L-Methionin, oder pharmazeutisch verträgliche Salze oder Ester davon und in beliebigen Kombinationen davon ist.

3. Zusammensetzung nach einem vorstehenden Anspruch, wobei
i) mindestens Riboflavin und D-Biotin umfasst, vorzugsweise i) Thiaminhydrochlorid, Riboflavin, Nicotinamid, D-Calciumpantothenat, Pyridoxinhydrochlorid, Cyanocobalamin, D-Biotin und Folsäure umfasst;
ii) L-Cystein-HCl-Monohydrat ist;
iii) L-Lysinmonohydrochlorid ist;
iv) eine aus Apfel extrahierte Procyanidin-B-2-Fraktion ist;
v) Mangostin ist;
vi) L-Methionin ist; und/oder
vii) hydrolysiertes Kollagen aus Fisch oder beliebige Kombinationen davon ist.

4. Zusammensetzung nach einem vorstehenden Anspruch, wobei eine Kollagenkomponente vii) in einer Menge von 40-80 Gew.-%, vorzugsweise 50-70 Gew.-%, basierend auf dem Gesamtgewicht der Zusammensetzung, vorliegt.

5. Zusammensetzung nach einem vorstehenden Anspruch, wobei die Summe von Komponenten i)-vi) 5-50 Gew.-% der Zusammensetzung, wie beispielsweise 10-30 Gew.-% der Zusammensetzung ausmacht.

6. Zusammensetzung nach einem vorstehenden Anspruch, wobei die Summe von Komponenten i)-vii) 60-90 Gew.-% der Zusammensetzung, wie beispielsweise 65-85 Gew.-%, z. B. 70-80 Gew.-% ausmacht.

7. Zusammensetzung nach einem vorstehenden Anspruch, wobei die Zusammensetzung in Pulverform vorliegt.

8. Nicht-therapeutische Verwendung einer Zusammensetzung nach den Ansprüchen 1-7 als Haarnachwuchsprodukt.

9. Zusammensetzung nach den Ansprüchen 1-7 zur Verwendung bei der Behandlung oder Vorbeugung von Haarausfall.

10. Zusammensetzung zur Verwendung nach Anspruch 9, wobei der Haarausfall durch Alopecia areata, eine Krebsbehandlung (wie Chemotherapie), Eisenmangel, eine Nebenwirkung eines Medikaments oder Gewichtsverlust verursacht wird.

11. Zusammensetzung zur Verwendung nach Anspruch 9, umfassend die Verabreichung der Zusammensetzung an einen Menschen in einer Dosierung von 2-10 g/Tag, wie beispielsweise 4-8 g/Tag.

## Revendications

1. Composition comprenant
i) riboflavine (vitamine B2) et biotine (vitamine B7) ;
ii) cystéine ;
iii) lysine ;
iv) procyanidine B-2 ;
v) extrait de mangoustan ;
vi) méthionine ; et
vii) collagène ;
viii) acide ascorbique ;
ix) acétate d'alpha-tocophéryle ;
x) gluconate de zinc ;
xi) fumarate ferreux ;
xii) gluconate de cuivre ;
xiii) bêta-carotène ;
xiv) chlorhydrate de thiamine ;
xv) nicotinamide ;
xvi) D-pantothénate de calcium ;
xvii) chlorhydrate de pyridoxine ;
xviii) cyanocobalamine ;
xix) acide folique ;
xx) L-sélénométhionine ; et
xxi) carbonate d'hydroxyde de magnésium ;
ou tout sel ou ester pharmaceutiquement acceptable de ceux-ci ;
dans laquelle la lysine est présente en une quantité de 5-12 % en poids sur la base du poids total de la composition ;
dans laquelle la procyanidine B-2 est présente en une quantité de 0,5-15 % en poids sur la base du poids total de la composition ;
dans laquelle la méthionine est présente en une quantité de 0,2-3,0 % en poids sur la base du poids total de la composition.

2. Composition selon la revendication 1, dans laquelle une ou plusieurs des exigences suivantes s'appliquent :
ii) est de la L-cystéine, iii) est de la L-lysine, et/ou vi) est de la L-méthionine, ou tout sel ou ester pharmaceutiquement acceptable de celles-ci, et dans une combinaison quelconque de ceux-ci.

3. Composition selon une quelconque revendication précédente, dans laquelle
i) comprend au moins de la riboflavine et de la D-biotine, de préférence i) comprend du chlorhydrate de thiamine, de la riboflavine, du nicotinamide, du D-pantothénate de calcium, du chlorhydrate de pyridoxine, de la cyanocobalamine, de la D-biotine et de l'acide folique ;
ii) est du monohydrate de L-cystéine HCl ;
iii) est du monochlorhydrate de L-lysine ;
iv) est une fraction de procyanidine B-2 extraite de la pomme ;
v) est de la mangostine ;
vi) est de la L-méthionine ; et/ou
vii) est du collagène hydrolysé provenant de poisson, ou toute combinaison de ceux-ci.

4. Composition selon une quelconque revendication précédente, dans laquelle le composant collagène vii) est présent en une quantité de 40-80 % en poids, de préférence 50-70 % en poids, sur la base du poids total de la composition.

5. Composition selon une quelconque revendication précédente, dans laquelle la somme des composants i)-vi) représente 5-50 % en poids de la composition, tel que 10-30 % en poids de la composition.

6. Composition selon une quelconque revendication précédente, dans laquelle la somme des composants i)-vii) représente 60-90 % en poids de la composition, tel que 65-85 % en poids, par ex. 70-80 % en poids.

7. Composition selon une quelconque revendication précédente, dans laquelle ladite composition est sous forme de poudre.

8. Utilisation non thérapeutique d'une composition selon les revendications 1-7 en tant que produit de repousse de cheveux.

9. Composition selon les revendications 1-7 destinée à être utilisée dans le traitement ou la prévention de la perte de cheveux.

10. Composition destinée à être utilisée selon la revendication 9, dans laquelle la perte de cheveux est provoquée par l'alopécie areata, un traitement contre le cancer (tel que la chimiothérapie), une carence en fer, un effet secondaire d'un médicament ou une perte de poids.

11. Composition destinée à être utilisée selon la revendication 9, comprenant l'administration de la composition à un humain à raison de 2-10 g/jour, tel que 4-8 g/jour.
